# EUROPÄISCHE PATENTSCHRIFT

(11) **EP 0 513 056 B1**
(45) Veröffentlichungstag und Bekanntmachung des Hinweises auf die Patenterteilung: **29.11.1995**
(21) Anmeldenummer: 91902701.1
(22) Anmeldetag: 25.01.1991
(51) Int. Cl.: C07D 215/18, C07D 215/48

(54) **VERFAHREN ZUR REINIGUNG VON 7-CHLOR-CHINOLIN-8-CARBONSÄUREN**
PROCESS FOR PURIFYING 7-CHLOR-QUINOLINE-8-CARBOXYLIC ACIDS
PROCEDE DE PURIFICATION D'ACIDES CARBOXYLIQUES DE 7-CHLORE-QUINOLEINE-8

(30) Priorität: 03.02.1990 DE 4003174
(43) Veröffentlichungstag der Anmeldung: 19.11.1992
(73) Patentinhaber: BASF Aktiengesellschaft, 67063 Ludwigshafen (DE)
(72) Erfinder: REISSENWEBER, Gernot, D-6737 Boehl-Iggelheim (DE); KOOB, Knut, D-6704 Mutterstadt (DE); RICHARZ, Winfried, D-6081 Stockstadt (DE)
(86) Internationale Anmeldenummer: EP9100143
(87) Internationale Veröffentlichungsnummer: WO9111436

(56) Entgegenhaltungen:
- EP-A- 0 085 182
- EP-A- 0 277 631
- EP-A- 0 282 778

## Beschreibung

Die vorliegende Erfindung betrifft ein verbessertes Verfahren zur Reinigung von 7-Chlor-chinolin-8-carbonsäuren der allgemeinen Formel I 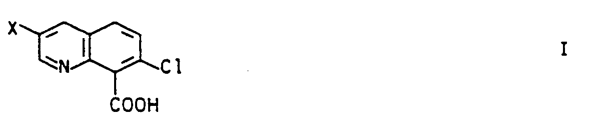
in der X Wasserstoff, Halogen oder eine niedere Alkylgruppe bedeutet.

Es ist allgemein bekannt (z.B. EP-A 085 182, EP-A 277 631 und EP-A 282 778), daß die als Wirkstoffe auf dem Pflanzenschutzgebiet dienenden Verbindungen vom Typ I durch Oxidation entsprechender 8-Methyl-, 8-Halogenmethyl- oder 8-Dihalogenmethylverbindungen mit Salpetersäure hergestellt werden können. Die wasserfeuchten Rohprodukte werden üblicherweise durch Umkristallisation aus niederen Alkoholen wie Methanol, Isopropanol (EP-A 277 631, EP-A 282 778) oder Methylglykol (EP-A 060 429) gereinigt.

Nachteilig bei diesen Reinigungsverfahren ist jedoch, daß die Chinolincarbonsäuren durch das alkoholische Lösungsmittel zum Teil verestert werden, was zu Carbonsäureverlusten von bis zu ca. 10 % führen kann.

Der Erfindung lag daher die Aufgabe Zugrunde, diesem Mangel abzuhelfen.

Demgemäß wurde ein Verfahren zur Reinigung von 7-Chlor-chinolin-8-carbonsäuren der Formel I gefunden, welches dadurch gekennzeichnet ist, daß man die Umkristallisation in Gegenwart von 0,1 bis 1 Gew.-%, bezogen auf die zu reinigende 7-Chlor-chinolin-8-carbonsäure, einer Base vornimmt.

Als Basen eignen sich in erster Linie die Alkalimetall- und Erdalkalimetallhydroxide, jedoch kommen auch die Alkalimetall- und Erdalkalimetallsalze von schwachen Säuren, beispielsweise Kohlensäure und Essigsäure sowie hochsiedende tertiäre Amine wie Triisobutylamin in Betracht.

Unter den Alkalimetall- und Erdalkalimetallhydroxiden werden die Alkalimetallhydroxide, insbesondere Natrium- und Kaliumhydroxid, besonders bevorzugt.

Die Base kann in fester Form oder als wäßrige Lösung eingesetzt werden.

Im allgemeinen ist es ausreichend, 0,1 bis 1 Gew.-%, insbesondere 0,2 bis 0,6 Gew.-% an Base, bezogen auf die zu reinigende (wasserfeuchte) Chinolincarbonsäure, einzusetzen.

Als Lösungsmittel für die Umkristallisation eignen sich vornehmlich mit Wasser mischbare niedere Alkohole mit bis zu 4 C-Atomen, darunter z.B. Alkanole wie Methanol, Ethanol, Isopropanol und Alkoxyalkanole wie Methylglykol und vor allem 1-Methoxypropan-2-ol.

Die Menge des verwendeten Lösungsmittels ist so zu bemessen, daß sich die zu reinigende Chinolincarbonsäure (Rohsubstanz) beim Erhitzen vollständig löst. Häufig empfiehlt es sich, die doppelte bis 10-fache Menge an Lösungsmittel, bezogen auf das Gewicht der wasserfeuchten Rohsubstanz I, zu verwenden.

Bei Verwendung von 1-Methoxypropan-2-ol als Lösungsmittel wird im allgemeinen etwa die 2- bis 6-fache Menge, bezogen auf das Gewicht der wasserfeuchten Rohsubstanz I, benötigt.

Sieht man von der erfindungsgemäßen Verbesserung ab, so wird die Umkristallisation wie üblich vorgenommen, indem man die wasserfeuchte, zu reinigende Chinolincarbonsäure I unter starkem Erwärmen und Rühren zusammen mit der Mineralbase in dem alkoholischen Lösungsmittel löst und die Lösung dann langsam bis auf etwa 20 bis 25°C abkühlen läßt. Um langsames Kristallwachstum und damit eine hohe Reinheit der kristallinen Endprodukte zu erreichen, empfiehlt sich ein linearer Abkühlgradient von etwa 10 bis 20°C/h. Die abgeschiedene kristalline Chinolincarbonsäure wird in üblicher Weise, z.B. durch Filtrieren oder Zentrifugieren abgetrennt und kann anschließend mit geringen Mengen an frischem, eventuell eisgekühltem Lösungsmittel von oberflächlichen Verunreinigungen aus der Mutterlauge freigewaschen werden. Eine besonders zweckmäßige Ausführungsform besteht darin, die zu reinigende Rohsubstanz I bei Temperaturen über 100°C in Lösung zu bringen. Verwendet man ein Lösungsmittel, das unterhalb dieser Temperatur siedet, so nimmt man das Verfahren im geschlossenen Gefäß unter dem Eigendruck der Lösung - bei etwa 3 bis 4 bar - vor. Die Methode empfiehlt sich besonders im Falle von 2-Methoxypropan-2-ol als Lösungsmittel. Dabei ist es vorteilhaft, die Rohsubstanz bei einer Temperatur von 130 bis 170°C zu lösen.

Das erfindungsgemäß verbesserte Verfahren läßt sich mit Erfolg auf alle definitionsgemäßen Chinolincarbonsäuren I anwenden, vor allem auf solche Verbindungen, bei denen X die folgende Bedeutung hat
- Wasserstoff;
- Halogen, bevorzugt Fluor, Chlor und Brom, insbesondere Chlor oder
- C₁-C₄-Alkyl wie Methyl, Ethyl, Propyl, Isopropyl, Butyl, sec.-Butyl und tert.-Butyl, vorzugsweise Methyl.

Die unerwünschten Verunreinigungen der Rohsubstanzen resultieren hauptsächlich aus der Bildung von Nebenprodukten sowohl bei der Chlorierung der als Ausgangssubstanz dienenden 8-Methylchinolincarbonsäure am Ringsystem und an der Methylgruppe, als auch bei der anschließenden Oxidation dieser Verbindungen Zu den Chinolin-8-carbonsäuren.

Der Wassergehalt der zu reinigenden 7-Chlor-chinolin-8-carbonsäuren liegt herstellungsbedingt im allgemeinen zwischen 1 und 50 %.

Die nach dem erfindungsgemäßen Verfahren mit höheren Ausbeuten zu reinigenden 7-Chlor-chinolin-8-carbonsäuren dienen beispielsweise als Pflanzenschutzmittel.

### Beispiele

Eine Suspension von jeweils 100 g einer wasserfeuchten, verunreinigten 7-Chlor-chinolin-8-carbonsäure I (Rohprodukt) in 400 g 1-Methoxypropan-2-ol wurde mit 1 g 50 %iger wäßriger Natronlauge versetzt und in einer geschlossenen Apparatur auf 150°C erhitzt, wobei sich ein Überdruck von ca. 3,1 bar einstellte. Nach vollständigem Auflösen der Carbonsäure ließ man im Verlauf von ca. 7 Stunden auf etwa 25°C abkühlen und filtrierte anschließend das auskristallisierte Produkt ab. Es wurde mit 100 ml Lösungsmittel nachgewaschen und getrocknet.

Zum Vergleich wurden diese Umkristallisationen unter identischen Bedingungen, aber ohne Zusatz einer Base, wiederholt.

Die Reinheit des umkristallisierten Produktes betrug jeweils etwa 97 bis 98 %.

Die folgende Tabelle zeigt für Chinolincarbonsäuren I mit unterschiedlichem Gehalt an Wasser und Verunreinigungen (= Rohprodukt Rw) den wesentlich geringeren Verlust an Wertprodukt bei Zugabe von Alkalimetallhydroxid.

In der Tabelle bedeuten:
- W: den Wassergehalt des Rohproduktes Rw
- Rg: das getrocknete Rohprodukt, ohne Wasseranteil
- I/Rg: den Anteil an Chinolincarbonsäure I am getrockneten Rohprodukt Rg (ohne Wasseranteil).

**Tabelle**

| Verlust an Chinolincarbonsäure I bei Umkristallisation aus 1-Methoxypropan-2-ol mit und ohne Zusatz von Natronlauge. | | | | | |
|---|---|---|---|---|---|
| Bsp. | X | W [Gew.-%] | I/Rg [Gew.-%] | Verlust an I bei der Umkristallisation [Gew.-%] | |
| | | | | mit NaOH | ohne NaOH |
| 1 | Cl | 39 | 76 | 12 | 24 |
| 2 | Cl | 32 | 81 | 12 | 24 |
| 3 | Cl | 31 | 83 | 9 | 19 |
| 4 | Cl | 24 | 66 | 12 | 20 |
| 5 | CH₃ | 30 | 77 | 13 | 18 |

## Patentansprüche

1. Verfahren zur Reinigung von 7-Chlor-chinolin-8-carbonsäuren der allgemeinen Formel I 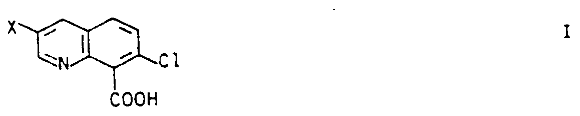 in der X Wasserstoff, Halogen oder eine niedere Alkylgruppe bedeutet, durch Umkristallisation aus mit Wasser mischbaren niederen Alkohlen, dadurch gekennzeichnet, daß man die Umkristallisation in Gegenwart von 0,1 bis 1 Gew.-%, bezogen auf die zu reinigende 7-Chlor-chinolin-8-carbonsäure, einer Base vornimmt.

2. Verfahren nach Anspruch 1, dadurch gekennzeichnet, daß man als Base ein Alkalimetall- oder Erdalkalimetallhydroxid verwendet.

3. Verfahren nach Anspruch 1 oder 2, dadurch gekennzeichnet, daß man 1-Methoxypropan-2-ol als Lösungsmittel verwendet.

4. Verfahren nach Anspruch 3, dadurch gekennzeichnet, daß man die umzukristallisierenden Chinolincarbonsäuren bei 130 bis 170°C unter dem Eigendruck des Systems in Lösung bringt.

## Claims

1. Process for purifying 7-chloroquinoline-8-carboxylic acids of the general formula I 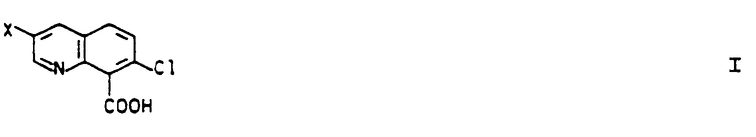 in which X denotes hydrogen, halogen or a lower alkyl group, by recrystallization from lower alcohols which are miscible with water, characterized in that the recrystallization is carried out in the presence of 0.1 to 1% by weight, based on the 7-chloroquinoline-8-carboxylic acid to be purified, of a base.

2. Process according to claim 1, characterized in that an alkali metal or alkaline earth metal hydroxide is used as base.

3. Process according to claim 1 or 2, characterized in that 1-methoxy-2-propanol is used as solvent.

4. Process according to claim 3, characterized in that the quinolinecarboxylic acids to be recrystallized are dissolved at 130 to 170°C under the autogenous pressure of the system.

## Revendications

1. Procédé d'épuration d'acides 7-chloro-quinolin-8-carboxyliques de formule générale I 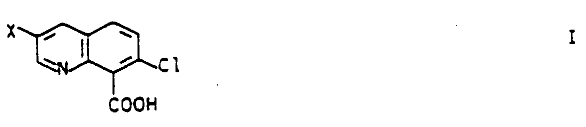 dans laquelle X représente hydrogène, halogène ou un groupe alkyle inférieur, par recristallisation d'alcools inférieurs miscibles avec l'eau, caractérisé par le fait que l'on effectue la recristallisation en présence de 0,1 à 1 % en poids, rapporté à l'acide 7-chloro-quinolin-8-carboxylique à épurer, d'une base.

2. Procédé selon la revendication 1, caractérisé par le fait que l'on utilise, comme base, un hydroxyde de métal alcalin ou alcalino-terreux.

3. Procédé selon la revendication 1 ou 2, caractérisé par le fait que l'on utilise le 1-méthoxypropane-2-ol comme solvant.

4. Procédé selon la revendication 3, caractérisé par le fait que l'on met en solution les acides quinolincarboxyliques à recristallisation, à 130 à 170°C, sous la pression propre du système.
